# EUROPEAN PATENT SPECIFICATION

(11) **EP 2 285 442 B1**
(45) Date of publication and mention of the grant of the patent: **02.01.2019**
(21) Application number: 09763304.4
(22) Date of filing: 03.06.2009
(51) Int. Cl.: A61M 25/00, A61M 31/00, A61B 5/083

(54) **SYSTEMS FOR ASSESSING LUNG FUNCTION AND DELIVERING THERAPEUTIC AGENTS**
SYSTEME ZUM TESTEN DER LUNGENFUNKTION UND ZUM AUSGEBEN VON HEILMITTELN
SYSTÈMES POUR ÉVALUER LA FONCTION PULMONAIRE ET ADMINISTRER DES AGENTS THÉRAPEUTIQUES

(30) Priority: 12.06.2008 US 60874 P
(43) Date of publication of application: 23.02.2011
(73) Proprietor: Pulmonx Corporation, Redwood City, CA 94063 (US)
(72) Inventor: ALJURI, Nikolai, Hillsborough CA 94010 (US); BEYHAN, Niyazi, Santa Clara CA 95051 (US)
(74) Representative: Clark, Jane Anne
(86) International application number: PCT/US2009/046138
(87) International publication number: WO 2009/152013

(56) References cited:
- US-A- 5 193 544
- US-A1- 2003 051 733
- US-A1- 2003 181 797
- US-A1- 2004 258 772
- US-A1- 2005 005 936
- US-A1- 2007 142 742

## Description

### BACKGROUND OF THE INVENTION

1. Field of the Invention. This invention relates generally to systems for assessing the functionality of lung compartments and treating diseased compartments of the lung.
2. Description of the Background Art. Lung diseases are a problem affecting several millions of people. Chronic obstructive pulmonary disease (COPD), for example, is a significant medical problem affecting 16 million people or about 6% of the U.S. population. Lung cancer, as another example, is among the most prevalent forms of cancer, and causes more than 150,000 deaths per year. In general, two types of diagnostic tests are performed on a patient to determine the extent and severity of lung disease: 1) imaging tests and 2) functional tests. Imaging tests, such as chest x-rays, computed tomography (CT) scans, Magentic Resonance Imaging (MRI), perfusion scans, and bronchograms, provide a good indicator of the location, homogeneity and progression of the diseased tissue. However, these tests do not give a direct indication of how the disease is affecting the patient's overall lung function and respiration capabilities. This can be measured with functional testing, such as spirometry, plethysmography, oxygen saturation, and oxygen consumption stress testing, to name a few. Together, these diagnostic tests are used to determine the course of treatment for the patient.

Further, the diagnostic tests for COPD are limited in the amount and type of information that may be generated. For example, diagnostic imaging may provide information to the physician regarding which lung regions "appear" more diseased, but in fact a region that appears more diseased may actually function better than one that appears less diseased. Functional testing is performed on the lungs as a whole. Thus, the information provided to the physician is generalized to the whole lung and does not provide information about functionality of individual lung compartments, which may be diseased. Thus, physicians may find it difficult in targeting interventional treatments to the compartments most in need and to avoid unnecessarily treating compartments that are not in need of treatment or less in need. In general, using conventional imaging or functional testing, the diseased compartments cannot be differentiated, prioritized for treatment or assessed after treatment for level of response to therapy.

Treatment for such lung diseases may include a variety of options. One such option is Lung Volume Reduction (LVR) which typically involves resecting diseased portions of the lung. Resection of diseased portions of the lungs both promotes expansion of the non-diseased regions of the lung and decreases the portion of inhaled air which goes into the lungs but is unable to transfer oxygen to the blood. Lung reduction is conventionally performed in open chest or thoracoscopic procedures where the lung is resected, typically using stapling devices having integral cutting blades. While effective in many cases, conventional lung reduction surgery is significantly traumatic to the patient, even when thoracoscopic procedures are employed. Such procedures often result in the unintentional removal of relatively healthy lung tissue or leaving behind of relatively diseased tissue, and frequently result in air leakage or infection.

An emerging method of lung volume reduction involves the endoscopic introduction of implants into pulmonary passageways. Such a method and implant is described in U.S. Patent Serial No 11/682,986. The implants will typically restrict air flow in the inhalation direction, causing the adjoining lung compartment to collapse over time. This method has been suggested as an effective approach for treating lung compartments that are not subject to collateral ventilation.

In the case of lung cancer treatment, other treatment options are available in addition to surgically resecting portions of the lung. One of the more common forms is chemotherapy, which can be either systemic or regional. Systemic chemotherapy is the oral or venous introduction of the chemotherapeutic agents into the body. Regional chemotherapy is the introduction of chemotherapeutic agents to the lung specifically, for example via inhalation or aerosol delivery. However, each of these therapies has their limitations. With each of these delivery methods, the medications are typically not introduced directly into the diseased portions of the lung. When administered systemically, the drugs reach many other areas of the body, and delivery to the pulmonary site of the cancer is inexact. Even when administered via inhalation or aerosol delivery, the treatment is generalized to several parts of the lung, and delivery to the specific site of cancer is inexact. Thus, these methods provide ineffective delivery of therapeutic agents, while having all the attendant problems of undesirable side-effects

An emerging cancer therapy is the introduction of anti-angiogenesis agents to prevent or eliminate the blood vessels that feed tumors. However, systemic or regional delivery of such agents is also subject to the limitations noted above.

Radiation therapy is also available as a treatment for lung cancer. However, the radiation is typically administered from the outside of the body, and not directly to the internal site of the disease. Thus, radiation therapy will affect non-diseased portions of the lungs, again causing undesirable side-effects. Additionally, radiation therapy is also not localized to the site of the cancer. Internal radiation therapy (commonly known as brachytherapy) involves the implantation of a radiation capsule near the tumor site, or the temporary intracavitary introduction of a radioactive element to the tumor site. In the case of lung cancer, the radioactive element can only be introduced into that portion of the lung large enough to accommodate the element.

For these reasons, it would be desirable to provide systems, methods, devices and kits which would overcome at least some of the shortcomings discussed above. First, it would be desirable to provide systems and methods of treatment targeted to a specific portion of a lung, where it will be most useful, and where it is less likely to cause unwanted side-effects. It would also be desirable to provide quantities or types of drugs that would be undesirable if provided globally. Second, it would be desirable to provide systems and methods for monitoring, assessing or measuring the functional state of individual lung compartments. It would be further desirable to provide systems and methods of comparing measured data of individual lung compartments to other individual lung compartments, to measured data of the lung as a whole, or to pre-obtained data of that patient's prior lung function, or to a range of data within a population. In addition, it would be desirable to provide systems and methods of estimating or predicting the outcome of treatment options prior to actual treatment, and also to assess the state of disease and functionality posttreatment. At least some of these objectives will be met by the inventions described hereinafter.

US 2003/0051733 describes systems, methods, devices and kits for assessing the level of pulmonal disease in individual lung compartments. An oxygen and/or carbon dioxide sensor may be provided. Oxygen sensors may be used in the performance of oxygen wash-out tests where the air in a lung compartment is replaced as much as possible with 100% oxygen.

US 2003/0181797 describes methods and a system for the transbronchial reflectance oximetric measurement of mixed venous oxygen saturation in the pulmonary artery of a patient.

US 2005/0005936 describes methods, systems and devices for improving ventilation in a lung area in which specific lung areas are ventilated with an indwelling trans-tracheobronchial catheter for the purpose of improving ventilation and reducing hyperinflation in that lung area and for redistributing inspired air to other healthier lung areas for treating respiratory disorders such as COPD, ARDS, SARS, CF and TB.

### BRIEF SUMMARY OF THE INVENTION

The present invention is set out in the appended claims. Described herein are methods and systems for assessing lung
compartment function, and treating diseased portions of the lung. In one embodiment, a method for assessing lung compartment function is disclosed. Said method comprises accessing a lung compartment through an isolation catheter, delivering an oxygen-enriched breathing gas to a lung compartment over a measurement period to elevate the oxygen level therein, and monitoring blood oxygen saturation, exhaled carbon dioxide concentration, or any combination thereof to detect changes therein over the measurement period. Several lung compartments may be monitored sequentially or simultaneously, and analyzed. An increase in blood oxygen level during the measurement period indicates that the corresponding lung compartment has a non-negligible lung function. Further, differences in blood oxygen level or exhaled carbon dioxide level of the various lung compartments can be analyzed to determine each compartment's relative contribution to overall lung function. The lung compartment's functions can also be compared to the patient's prior history or to normal values in the population. An analyzed compartment may thereafter be treated, as appropriate. Such treatment may include delivering a therapeutic agent to the lung compartment.

Another embodiment described herein contemplates a method for treating a lung compartment that comprises isolating a lung compartment from the rest of a lung, and delivering a therapeutic agent to the isolated compartment for a predetermined period of time. Optionally, this treatment method further comprises monitoring the concentration of the therapeutic agent in the lung compartment. In one aspect, the therapeutic agent is carbon monoxide, wherein the carbon monoxide concentration in blood does not reach toxic levels. The carbon monoxide concentration may be monitored to prevent it from reaching toxic levels. The inhaled carbon monoxide in the isolated compartment acts as a broncho-dilating and anti-inflammatory or anti-oxidizing agent. In another aspect, the therapeutic agent is a radioactive gas in sufficient concentration to prevent or inhibit the proliferation of cancer cells. In yet another aspect, the therapeutic agent could be a chemotherapeutic agent in sufficient concentration to prevent or inhibit the proliferation of cancer cells. In yet another aspect, the therapeutic agent could be of an angiocidal nature, an anti-angiogenetic nature or any combination thereof to eliminate existing blood vessels inhibit new blood vessel formation.

### BRIEF DESCRIPTION OF THE DRAWINGS

The invention has other advantages and features which will be more readily apparent from the following detailed description of the invention and the appended claims, when taken in conjunction with the accompanying drawings, in which:
Figure 1 shows a diagram of an isolation catheter.
Figure 2 shows the isolation catheter accessing a lung compartment.
Figure 3 shows a diagram of a control unit.

### DETAILED DESCRIPTION OF THE INVENTION

Although the detailed description contains many specifics, these should not be construed as limiting the scope of the invention but merely as illustrating different examples and aspects of the invention. It should be appreciated that the scope of the invention includes other embodiments not discussed in detail above. Various other modifications, changes and variations which will be apparent to those skilled in the art may be made in the arrangement, operation and details of the systems of the present invention disclosed herein without departing from the scope of the claimed invention.

Described herein are methods and systems for targeting, accessing, diagnosing and treating diseased lung compartments; such compartments could be an entire lobe, a segment, a subsegment and beyond. Diagnosis is achieved in the present invention by isolating a lung compartment to obtain compartment-specific measurements to determine the lung functionality. Treatment is achieved by isolating a lung compartment to provide targeted delivery of therapeutic agents to that region of the lung. Though COPD is mentioned as an example, the applicability of these methods for treatment and diagnosis is not limited to COPD, but includes any disease of the lung.

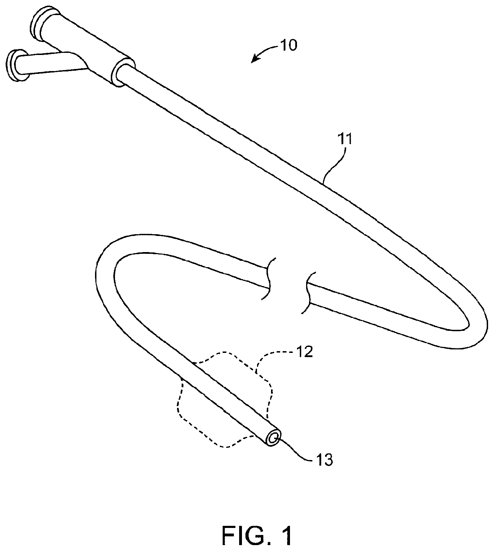

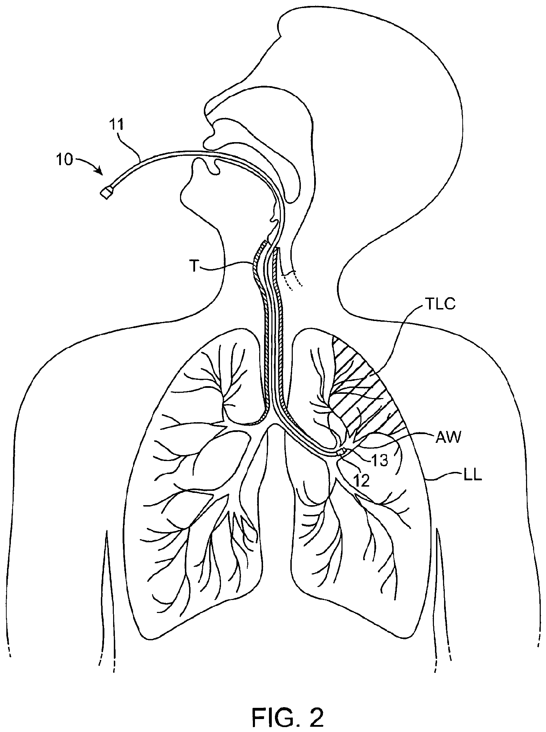

In the present embodiments, isolation of the lung comprises sealingly engaging a distal end of a catheter in an airway feeding a lung compartment, as shown in Figures 1 and 2. Such a catheter has been disclosed in co-pending U.S. Patent Publication No. 20080051719, As shown in Figure 1, the catheter 10 comprises a catheter body 11, an expandable occluding member 12 on the catheter body, and a mechanism for introducing gases or other agents into the lung. The catheter body 11 usually has a distal end, a proximal end, and at least one lumen 13 extending from a location at or near the distal end to a location at or near the proximal end. The expandable occluding member 12 is disposed near the distal end of the catheter body and is adapted to be expanded in the airway which feeds the target lung compartment.

At least a distal portion of the catheter body 11 is adapted to be advanced into and through the trachea (T), as shown in Figure 2. The distal end of the catheter 10 can then be directed to a lung lobe (LL) to reach an airway (AW) which feeds a target lung compartment (TLC), wherein the compartment is to be assessed and/or treated. When the occluding member 12 is expanded, that compartment is isolated with access provided through the lumen 13 or catheter body when the occluding member 12 is expanded.

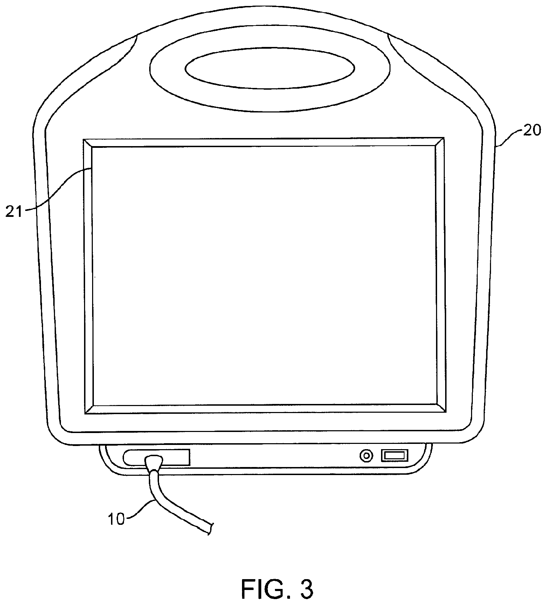

The proximal end of the catheter is associated with a control unit 20, as shown in Figure 3. The control unit 20 comprises mechanisms to measure lung functionality. Control unit 20 may also comprise mechanisms to introduce a gas or any agent into the isolated lung compartment via the catheter lumen. The control unit 20 will typically comprise an interface for receiving input from a user and a display screen 21. The display-screen 21 will optionally be a touch-sensitive screen, and will display preset values. Optionally, the user will input information into the control unit 20 via a touch-screen mechanism.

In one aspect, lung functionality is assessed by introducing an oxygen rich gas into an isolated lung compartment and measuring blood oxygen saturation throughout the body, carbon dioxide exhaled from the isolated compartment, or a combination thereof. A first lung compartment is isolated by the catheter 10 with an occluding member as described above. Oxygen rich gas is then introduced into the lung through the lumen 13 of the catheter 10. Such oxygen rich gas may originate from one or more sources that may include the control unit 20, an external canister (not shown) or any other source. Subsequently to or simultaneously with the introduction of oxygen rich gas, blood oxygen saturation (hereinafter also referred to as oxygen saturation) is measured. For example, blood oxygen saturation can be monitored by a fingertip pulse oximeter, and communicated to control unit 20. Additionally or alternatively, carbon dioxide levels in exhalation (hereinafter also referred to as carbon dioxide levels) through the catheter lumen may be monitored by one or more sensors. Such sensors may reside within the control unit 20 and inline with the catheter, or they may reside within the catheter 10 itself, or they may reside elsewhere and inline with the catheter 10.

Control unit 20 assesses the values obtained from monitoring blood oxygen and/or carbon dioxide exhalation levels to determine the isolated lung compartment's function either singularly or comparatively. Singular lung function can be determined by noting any change in global blood oxygen level or exhaled carbon dioxide level during assessment of a single compartment. An increase in global blood oxygen or exhaled compartmental carbon dioxide level during the measurement time would indicate that the corresponding lung compartment is significantly and positively contributing to overall gas exchange in the lung, and thus has a non-negligible lung function. Optionally, the measurement values thus obtained could be assessed against previously obtained values, such as the patient's own medical history, or normal values in a population segment.

As an optional step, a second lung compartment is isolated by a catheter, and the corresponding blood oxygen saturation, carbon dioxide exhalation level or any combination thereof, is monitored. The process could be repeated for several compartments of a lung. The measurements could be obtained sequentially, wherein values from the lung compartments are obtained one at a time, or the measurements could be obtained simultaneously, wherein values from several lung compartments are obtained at the same time via multiple catheters placed in several lung compartments simultaneously.

Further, the lung compartments could be comparatively assessed to determine the relative functionality of each evaluated lung compartment. After measurements are obtained from multiple lung compartments, the control unit 20 compares each lung compartment's measurements to other lung compartment measurements. The control unit 20 thus determines the relative contribution of each of those compartments to overall respiration. The measurements may also be compared to prior patient history, or to normal population values.

Optionally, those lung compartments that contribute least to overall function, as determined by comparison to other lung compartments, to the patient's history, or to normal population values, are determined to be diseased. Additionally and optionally, any of the lung compartments assessed in the methods herein described, maybe combined with other methods for diagnosing lungs.

If a lung compartment is determined to be diseased, it is treated using the same isolation catheter described above. The target lung compartment is isolated by a catheter 10 with an occluding member 12, as described above, and therapeutic agents can thereafter be introduced directly into that lung compartment. Such therapeutic agents may originate from one or more sources that may include the control unit 20, an external canister (not shown) or any other source. Optionally, the concentration of the therapeutic agent in the lung compartment may be monitored to ensure that the appropriate concentrations are reaching the lung compartment.

In one aspect, such treatment of a lung compartment comprises isolating that compartment and delivering non-toxic levels of carbon monoxide to that compartment. For example, to treat an inflamed or constricted portion of the lung, carbon monoxide could be introduced directly into the lung compartment as a broncho-dilating and anti-inflammatory agent or an anti-oxidant agent in a concentration that is non-toxic to the patient, but sufficient to deter inflammation and promote broncho-dilation. In a second aspect, anti-cancer agents may be introduced into the lung. For example, in the case of a lung compartment that is determined to be cancerous, a radioactive gas, anti-angiogenesis or angiocidal agent, chemotherapeutic agent or any other substance could be targeted to a region by introduction through the catheter lumen 13.

This method of direct introduction ensures that the therapeutic agent is only delivered to those areas where it is needed. Simultaneously, isolated introduction of a therapeutic agent would be advantageous by ensuring proliferation of the agent throughout the entire section to be treated. Subsequently, such introduction minimizes the negative side effects associated with the systemic methods of treating a lung, while enhancing localized delivery of therapeutic agents.

Additionally or alternatively, concentration of the therapeutic agent in the isolated lung compartment may be monitored by one or more sensors. Such sensors may reside within the control unit 20, within catheter 10, in-line with the catheter 10, or elsewhere.

Additionally or alternatively, the access catheter may be used in conjunction with virtual bronchoscopy techniques, magnetic tracking techniques or any combination thereof thereby allowing access to pulmonary passageways that are too small for conventional bronchoscopes to navigate. For example, using virtual bronchoscopy techniques, such as those described in the above referenced patent applications, a user may create a virtual representation (such as a three dimensional model) of the patient's pulmonary pathways. Thereafter, the user can employ the virtual representation, rather than a bronchoscope, as a visualization and navigation tool to guide a catheter through a narrow passageway of the lung to the treatment site. Additionally or alternatively, the user can use electro-magnetic tracking to visualize the location of the catheter and guide it through the pulmonary passageways to the site of treatment.

While the above is a complete description of the preferred embodiments of the invention, various alternatives, modifications, and equivalents may be used. Therefore, the above description should not be taken as limiting the scope of the invention which is defined by the appended claims.

## Claims

1. A system for assessing lung compartment function, comprising:
an oxygen control unit (20) for delivering oxygen-enriched breathing gas to the lung compartment to elevate the oxygen level therein; and
an isolation catheter (10) configured to be attachable to the control unit (20), wherein the isolation catheter (10) is configured to isolate a lung compartment and wherein the oxygen control unit (20) is configured to deliver the oxygen-enriched breathing gas to the lung compartment using the isolation catheter (10);
wherein the control unit (20) is configured to monitor at least one of blood oxygen and exhaled carbon dioxide to produce an assessment of a lung compartment function by: a) determining a change in the monitored blood oxygen level or exhaled carbon dioxide level or b) determining a difference between the monitored blood oxygen level or exhaled carbon dioxide level against one or more previously obtained values;
**characterized in that** the isolation catheter (10) is configured to deliver one or more therapeutic agents to the isolated lung compartment based on the assessment of a lung compartment function by the control unit (20).

2. The system of claim 1, further comprising one or more sources of oxygen enriched breathing gas.

3. The system of claim 1, further comprising one or more sources of the therapeutic agent.

4. The system as in claim 1, wherein the therapeutic agent control unit monitors the concentration of the therapeutic agent.

5. The system as in any one of claims 1 to 4, wherein the therapeutic agent is carbon monoxide, and wherein the carbon monoxide is delivered such that the carbon monoxide concentration in blood does not reach toxic levels.

6. The system as in claim 5, wherein the inhaled carbon monoxide in the isolated compartment acts as an anti-inflammatory, broncho-dilating, or anti-oxidizing agent.

7. The system as in anyone of claims 1 to 4, wherein the therapeutic agent is a radioactive gas in sufficient concentration to prevent or inhibit the proliferation of cancer cells.

8. The system as in anyone of claims 1 to 4, wherein the therapeutic agent is a chemotherapeutic agent in sufficient concentration to prevent or inhibit the proliferation of cancer cells.

9. The system as in anyone of claims 1 to 4, wherein the therapeutic agent is an anti-angiogenesis agent, an angiocidal agent, or any combination thereof.

10. The system of anyone of claims 1 to 4, wherein the therapeutic agent is carbon monoxide, radioactive gas or chemotherapeutic agent.

## Patentansprüche

1. System zur Bewertung der Funktion eines Lungenkompartiments, umfassend:
eine Sauerstoff-Kontrolleinheit (20) zur Abgabe von mit Sauerstoff angereichertem Atemgas an das Lungenkompartiment zur Erhöhung des Sauerstoffgehalts darin; und
einen Isolationskatheter (10), der derart konfiguriert ist, dass er sich an der Kontrolleinheit (20) befestigen lässt, wobei der Isolationskatheter (10) zum Isolieren eines Lungenkompartiments konfiguriert ist und wobei die Sauerstoff-Kontrolleinheit (20) zur Abgabe des mit Sauerstoff angereicherten Atemgases mittels des Isolationskatheters (10) an das Lungenkompartiment konfiguriert ist;
wobei die Kontrolleinheit (20) konfiguriert ist zur Überwachung von mindestens einem von Blutsauerstoff und ausgeatmetem Kohlendioxid zum Erzielen einer Bewertung der Funktion eines Lungenkompartiments durch: a) Ermitteln einer Änderung des überwachten Sauerstoffgehalts im Blut oder des Gehalts an ausgeatmetem Kohlendioxid oder b) Ermitteln eines Unterschieds zwischen dem überwachten Sauerstoffgehalt im Blut oder dem Gehalt an ausgeatmetem Kohlendioxid gegen einen oder mehr zuvor erhaltene(n) Wert(e);
**dadurch gekennzeichnet, dass** der Isolationskatheter (10) konfiguriert ist zur Abgabe von einem oder mehr therapeutischen Mittel(n) an das isolierte Lungenkompartiment bezogen auf die Bewertung der Funktion eines Lungenkompartiments durch die Kontrolleinheit (20).

2. System nach Anspruch 1, weiter umfassend eine oder mehr Quelle(n) von mit Sauerstoff angereichertem Atemgas.

3. System nach Anspruch 1, weiter umfassend eine oder mehr Quelle(n) des therapeutischen Mittels.

4. System nach Anspruch 1, wobei die Kontrolleinheit für das therapeutische Mittel die Konzentration des therapeutischen Mittels überwacht.

5. System nach einem der Ansprüche 1 bis 4, wobei das therapeutische Mittel Kohlenmonoxid ist, und wobei das Kohlenmonoxid derart abgegeben wird, dass die Kohlenmonoxidkonzentration im Blut keine toxischen Spiegel erreicht.

6. System nach Anspruch 5, wobei das inhalierte Kohlenmonoxid in dem isolierten Kompartiment als ein entzündungshemmendes, bronchodilatierendes oder oxidationshemmendes Mittel wirkt.

7. System nach einem der Ansprüche 1 bis 4, wobei das therapeutische Mittel ein radioaktives Gas in ausreichender Konzentration zur Verhinderung oder Inhibition der Proliferation von Krebszellen ist.

8. System nach einem der Ansprüche 1 bis 4, wobei das therapeutische Mittel ein chemotherapeutisches Mittel in ausreichender Konzentration zur Verhinderung oder Inhibition der Proliferation von Krebszellen ist.

9. System nach einem der Ansprüche 1 bis 4, wobei das therapeutische Mittel ein Antiangiogenesemittel, ein angiozides Mittel oder jedwede Kombination davon ist.

10. System nach einem der Ansprüche 1 bis 4, wobei das therapeutische Mittel Kohlenmonoxid, radioaktives Gas oder ein chemotherapeutisches Mittel ist.

## Revendications

1. Système d'évaluation d'une fonction d'un compartiment pulmonaire, comprenant :
une unité de contrôle de l'oxygène (20) pour amener un gaz respiratoire enrichi en oxygène au compartiment pulmonaire afin d'y élever le niveau d'oxygène ; et
un cathéter d'isolement (10) configuré pour pouvoir être monté sur l'unité de commande (20), le cathéter d'isolement (10) étant configuré pour isoler un compartiment pulmonaire et l'unité de contrôle de l'oxygène (20) étant configurée pour amener le gaz respiratoire enrichi en oxygène au compartiment pulmonaire en utilisant le cathéter d'isolement (10) ;
dans lequel l'unité de commande (20) est configurée pour surveiller au moins l'un de l'oxygène sanguin et du dioxyde de carbone expiré pour produire une évaluation d'une fonction du compartiment pulmonaire, par : a) détermination d'un changement dans le taux d'oxygène sanguin ou le taux de dioxyde de carbone expiré qui fait l'objet de la surveillance, ou b) détermination d'une différence entre le taux d'oxygène sanguin ou le taux de dioxyde de carbone expiré qui fait l'objet de la surveillance par rapport à une ou plusieurs valeurs obtenues auparavant ;
**caractérisé en ce que** le cathéter d'isolement (10) est configuré pour amener un ou plusieurs agents thérapeutiques au compartiment pulmonaire isolé en fonction de l'évaluation d'une fonction du compartiment pulmonaire par l'unité de contrôle (20).

2. Système selon la revendication 1, comprenant en outre une ou plusieurs sources de gaz respiratoire enrichi en oxygène.

3. Système selon la revendication 1, comprenant en outre une ou plusieurs sources de l'agent thérapeutique.

4. Système selon la revendication 1, dans lequel l'unité de contrôle de l'agent thérapeutique surveille la concentration de l'agent thérapeutique.

5. Système selon l'une quelconque des revendications 1 à 4, dans lequel l'agent thérapeutique est le monoxyde de carbone, et dans lequel le monoxyde de carbone est amené de telle sorte que la concentration de monoxyde de carbone dans le sang n'atteigne pas des niveaux toxiques.

6. Système selon la revendication 5, dans lequel le monoxyde de carbone inhalé dans le compartiment isolé agit comme un agent anti-inflammatoire, bronchodilatateur, ou antioxydant.

7. Système selon l'une quelconque des revendications 1 à 4, dans lequel l'agent thérapeutique est un gaz radioactif en concentration suffisante pour prévenir ou inhiber la prolifération de cellules cancéreuses.

8. Système selon l'une quelconque des revendications 1 à 4, dans lequel l'agent thérapeutique est un agent chimiothérapeutique en concentration suffisante pour prévenir ou inhiber la prolifération de cellules cancéreuses.

9. Système selon l'une quelconque des revendications 1 à 4, dans lequel l'agent thérapeutique est un agent antiangiogenèse, un agent angiocide, ou une combinaison quelconque de ceux-ci.

10. Système selon l'une quelconque des revendications 1 à 4, dans lequel l'agent thérapeutique est le monoxyde de carbone, un gaz radioactif ou un agent chimiothérapeutique.
